# DEMANDE DE BREVET EUROPEEN

(11) **EP 1 195 147 A1**
(43) Date de publication de la demande: **10.04.2002**
(21) Numéro de dépôt: 02000785.2
(22) Date de dépôt: 14.04.1995
(51) Int. Cl.: A61F 2/01

(54) **Filtre sanguin endovasculaire et son dispositif d'implantation**

(30) Priorité: 21.04.1994 FR 9404804
(62) Demande divisionnaire de: 95400850.4
(71) Demandeur: B. Braun Medical SAS,(Société par Actions Simplifiées), 92100 Boulogne-Billancourt (FR)
(72) Inventeur: Chevillon, Gérard, 92120 Montrouge (FR); Nadal, Guy, 86000 Poitiers (FR); Iachetti, Massimo, Prof., 00199 Roma (IT)
(74) Mandataire: Lerner, François

(57) **Abrégé**

Il s'agit d'un ensemble utilisant un filtre (3₃) vasculaire logé dans une gaine (5) d'implantation dont il peut être sorti en partie dans une utilisation de filtration temporaire par l'intermédiaire d'une tige de manoeuvre (9₃) à laquelle le filtre est lié de manière séparable, de sorte qu'en utilisation définitive, le filtre en soit séparé, étant alors expulsé hors de la gaine.

Le filtre peut présenter des pattes (131₃) en corolle et des appendices (15₃) de maintien en place dans le vaisseau qui définissent naturellement un cylindre.

Application au traitement des risques d'embolie.

## Description

L'invention a pour objet un filtre médical destiné à être positionné dans un vaisseau sanguin pour piéger des caillots pouvant y circuler.

Pour implanter ces filtres dans le corps d'un patient, le praticien utilise habituellement la voie endoveineuse, en pratiquant soit une dénudation vasculaire soit une technique d'introduction dite percutanée.

La technique percutanée est la moins agressive. Après avoir pratiqué une incision de la peau, le filtre est introduit dans la veine retenue, puis est guidé jusqu'à la veine cave inférieure où il est "expulsé" hors du dispositif. Il s'expanse ainsi dans la veine, en prévenant la migration des caillots qui se dirigent vers le coeur et l'artère pulmonaire.

Suivant les risques, deux grandes catégories de filtres sont disponibles.

Face tout d'abord à des risques d'embolie graves, on propose des filtres dits "définitifs", c'est-à-dire prévus pour être implantés de façon permanente.

De tels filtres sont par exemple décrits dans les brevets US-A-3 540 431, US-A-4 688 553 ou encore dans FR-A-2 689 388.

Dans ces deux cas typiques, le filtre est constitué d'éléments filiformes disposés pour définir une corolle dont l'axe doit avantageusement être orienté sensiblement suivant l'axe du vaisseau, de manière à obtenir l'effet le meilleur de retenue des caillots, sans perturber exagérément la circulation du sang.

Etant implanté de façon définitive, ces filtres doivent être fixés à la veine et comportent souvent pour cela des crochets qui s'ancrent dans la paroi du vaisseau au moment où le filtre est lâché et s'expanse radialement.

Couramment, le dispositif d'implantation de ces filtres comprend une gaine s'étendant depuis l'extérieur du corps du patient et dans laquelle le filtre est retenu à l'état radialement contraint. Une tige de manoeuvre montée glissante dans la gaine permet d'expulser le filtre hors de cette gaine, laquelle est ensuite retirée avec la tige.

Il arrive toutefois que les risques d'embolie ne nécessitent pas un traitement de filtration in situ permanent.

On a donc déjà proposé des filtres dits "temporaires" (par exemple décrits dans FR-A-2 694 687 ou FR-A-2 657 261), c'est-à-dire présentant une structure telle qu'ils puissent être retirés après avoir été implantés.

Classiquement, il s'agit en fait d'instruments médicaux où l'on retrouve une gaine extérieure dans laquelle glisse une tige de manoeuvre à une extrémité de laquelle est fixé de manière définitive un moyen de filtration, lequel peut en particulier se présenter à nouveau comme une structure filiforme se développant naturellement suivant une corolle conique, laquelle est radialement contrainte dans la gaine tant que le filtre n'est pas opérant. La libération du filtre s'effectue une fois encore par déplacement relatif axial de la gaine et de la tige, permettant à la corolle de filtration de s'épanouir. Dans la mesure où les moyens de filtration sont dépourvus de crochet d'ancrage et sont tenus par la tige, on peut les retirer par la voie d'accès veineuse.

Ces filtres temporaires présentent l'inconvénient d'être parfois emprisonnés trop rapidement par les tissus du vaisseau récepteur. Ce problème peut aussi exister sur les filtres définitifs, notamment lorsqu'il s'avère nécessaire, après une certaine durée d'implantation, de repositionner le filtre dans le vaisseau, par exemple du fait d'un mauvais centrage.

L'invention apporte une solution à ce problème en augmentant la surface d'appui du filtre contre la paroi du vaisseau récepteur.

En particulier, les revendications jointes 1, 3, 5, définissent les caractéristiques qui y concourent.

Les revendications subsidiaires 2, 4, 6 et 7 recommandent l'utilisation de fil(s) plat(s) pour accroître cette surface d'appui.

Une description plus détaillée de l'invention suit, en référence aux dessins annexés dans lesquels :
- les figures 1 et 2 montrent deux variantes du filtre de l'invention,
- la figure 3 montre une troisième variante du filtre de l'invention, et
- la figure 4 montre, en vue agrandie et partielle, le filtre de la figure 3 disposé dans la gaine d'implantation, dans un état contraint radialement.

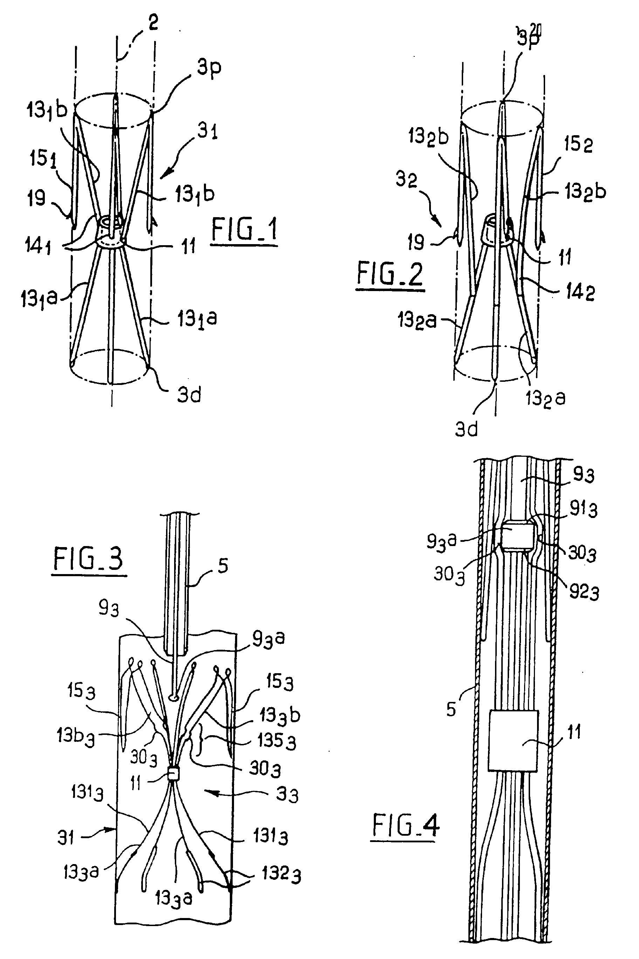

Sur les figures 1 et 2, une première variante 3₁ et une seconde variante 3₂ du filtre de l'invention sont représentées. Elles comprennent une partie de filtration sensiblement conique, dépourvue de moyens d'ancrage au vaisseau et formée par la tête 11 de laquelle sont issues des premières pattes 13₁a, 13₂a sensiblement rectilignes qui s'étendent jusque vers l'extrémité distale 3_{d} du filtre.

La tête 11 est pourvue de moyens de liaison avec une tige de manoeuvre montée axialement coulissante dans une gaine.

Les filtres 3₁ et 3₂ comprennent chacun une série de secondes pattes 13₁b et 13₂b dont certaines au moins sont prolongées par les appendices 15₁ et 15₂ pour le maintien en place et le centrage respectivement des filtres 3₁ et 3₂ dans le vaisseau. Ces appendices comportent des moyens de fixation 19 du filtre contre la paroi du vaisseau, ces crochets 19 étant écartés de l'extrémité distale 3_{d} du filtre. Les secondes pattes et leurs appendices présentent une forme générale en épingle à cheveux et sont formés d'une seule pièce.

D'après la figure 1, les pattes 13₁b du filtre 3₁ sont fixées, à une extrémité de fixation 14₁, à la partie filtrante (au niveau de la tête 11). Elles s'étendent depuis cette tête jusqu'à l'extrémité proximale 3ₚ du filtre, de telle sorte que, dans leur état radialement non contraint, elles définissent une seconde corolle sensiblement conique, la première corolle de filtration et la seconde corolle étant tête-bêche.

D'après la figure 2, chaque patte 13₂b est fixée sur une patte 13₂a, en une portion ou zone de cette patte écartée de ses extrémités opposées (par exemple sensiblement en son milieu) de telle sorte que, dans leur état non contraint, les pattes 13₂b s'étendent, depuis leur extrémité de fixation 14₂ aux pattes 13₂a, suivant une seconde corolle ou une surface tubulaire allant en s'évasant, d'axe 2 (qui entoure la tête 11).

Les périmètres d'ouverture des première et seconde corolles pourront être sensiblement égaux (la seconde corolle filtrant également le sang qui la traverse).

Dans l'état non contraint des moyens de maintien, les appendices 15₁ et 15₂ s'étendent, sur une partie au moins de leur longueur, sensiblement parallèlement à l'axe 2 en définissant une surface tubulaire de section sensiblement égale à celle du vaisseau pour que ces appendices viennent au contact de la paroi du vaisseau et y fixent le filtre. Ces appendices sont dirigés en direction de l'extrémité 3_{d} du filtre, ainsi la seconde corolle s'étend à l'intérieur de la surface tubulaire définie par les appendices.

En utilisation temporaire des filtres 3₁ et 3₂, les appendices porteurs des crochets 19, et également ici les secondes pattes, seront maintenus dans la gaine dans un état radialement contraint.

Les appendices et les secondes pattes sont constitués par des fils métalliques ronds ou plats, les secondes pattes étant par exemple soudées sur la première corolle.

Le filtre 3₃ de la figure 3 se distingue de celui de la figure 1 en particulier en ce que chaque seconde patte 13₃b et son appendice 15₃ sont réalisés, de préférence en une seule pièce, par un fil souple replié sur lui-même en boucle(s) et dont les deux extrémités opposées, rapprochées l'une de l'autres, sont fixées à la tête 11 (pour plus de détails sur la constitution de ces pattes 13₃b et de leurs appendices, on pourra se reporter à US 5 344 427). Quant à certaines au moins des pattes 13₃a, elles comportent une partie principale 131₃ en fil raccordée à la tête et qui se prolonge par une partie distale 132₃ à contour non agressif vis-à-vis du vaisseau 31 et de section supérieure à celle du fil pour augmenter la surface d'appui du filtre contre la paroi en ralentissant l'emprisonnement de ces parties distales par les tissus du vaisseau.

De plus, le filtre 3₃ se distingue respectivement des filtres 3₁ et 3₂ en ce que les moyens de liaison amovible comprennent ici des moyens de serrage, ou de blocage axial, de la tige qui sont prévus sur les secondes pattes 13₃b (filtre 3₃) et 13₄b (filtre 3₄) en une zone 135₃, 135₄ intermédiaire entre la tête 11 et l'extrémité proximale du filtre (donc écartée des extrémités opposées du filtre). Ces moyens de serrage coopèrent avec des moyens complémentaires de la tige qui comprennent au moins une butée coopérante. Figure 3, les appendices 15₃ sont, seulement sur une partie de leur longueur, parallèles à l'axe 2.

A la figure 4, la tige 9₃ se termine par une extrémité 9₃a renflée qui présente deux butées 91₃ et 92₃, certaines au moins des pattes 13₃b présentant localement une portion recourbée ou petite boucle ouverte 30₃ pour le logement de cette extrémité renflée. Dans l'état contraint des moyens de maintien, lorsqu'ils sont contenus dans la gaine (utilisation temporaire du filtre), les portions 30₃ enserrent ou emprisonnent l'extrémité 9₃a de la tige, en venant en appui contre les butées pour empêcher tout déplacement axial de la tige par rapport au filtre. Dans l'utilisation définitive du filtre, les moyens de serrage, hors de la gaine, sont écartés de la butée du fait de l'expansion radiale des secondes pattes.

On notera encore d'après les figures 3 et 4 que la tige présente ici un faible diamètre extérieur par rapport au diamètre intérieur de la gaine, de telle sorte que les secondes pattes et leurs appendices soient disposés entre la tige et la gaine dans l'utilisation temporaire du filtre.

Concernant le type de filtre visé dans l'invention, on notera qu'il pourrait aussi s'agir du filtre à corolle unique de la demande FR-A-2 694 491 (filtre à pattes triangulées) ou encore de celui de la demande FR-A-2 699 809 avec des appendices conformés en zigzag.

Pour la technique générale d'implantation du filtre concerné, on pourra se reporter en particulier aux publications précitées, ou encore au brevet US-A-4 990 156, étant précisé qu'après avoir ménagé une incision, on forme une voie d'accès jusqu'à la zone d'implantation du filtre, puis, on introduit la gaine introductrice 5 pour qu'elle s'étende le long de la voie d'accès, son extrémité proximale ressortant du corps du patient tandis que son extrémité distale est dans le vaisseau d'implantation. En l'espèce, la mise en place du filtre, dans un état contraint, s'effectue ensuite à partir de l'extrémité proximale de la gaine, le filtre étant poussé le long de ce cathéter par la tige (telle que 9₃) liée au filtre jusqu'à ce qu'il soit positionné dans l'extrémité distale de la gaine.

Pour la mise en place du filtre, on pourrait éventuellement utiliser un cathéter supplémentaire dans lequel pourrait glisser la gaine, de manière à faciliter son introduction.

## Revendications

1. Filtre sanguin expansible dans un vaisseau (31) d'un patient, sensiblement radialement vis-à-vis de l'axe dudit vaisseau, pour y retenir d'éventuels caillots de sang, le filtre comprenant une tête (11) de laquelle sont issues des pattes (13₃a) expansibles radialement suivant sensiblement une corolle, dans un état radialement non contraint,
**caractérisé en ce que** certaines au moins desdites pattes comportent une partie principale (131₃) en fil raccordée à la tête (11) et qui se prolonge par une partie distale (132₃) de section supérieure à celle de la partie principale, pour augmenter la surface d'appui du filtre contre la paroi du vaisseau.

2. Filtre selon la revendication 1, **caractérisé en ce que** ladite partie distale des pattes forme un appendice constitué par un fil métallique plat.

3. Filtre sanguin expansible dans un vaisseau (31) d'un patient, sensiblement radialement vis-à-vis de l'axe dudit vaisseau, pour y retenir d'éventuels caillots de sang, le filtre comprenant :
-- des moyens de filtration qui comportent une tête (11) de laquelle sont issues des pattes (13₁b ; 13₃a, 13₃b) expansibles radialement suivant sensiblement au moins une corolle, dans un état radialement non contraint,
-- des moyens (15, 15₁, 15₂, 132₃) de maintien en place du filtre par rapport au vaisseau, ces moyens de maintien en place étant liés aux moyens de filtration et comprenant des appendices (132₃, 15₁, 15₂) qui prolongent une partie principale (131₃) de certaines au moins desdites pattes des moyens de filtration, qui viennent au contact du vaisseau,
**caractérisé en ce que** la partie principale (131₃) de certaines au moins des pattes est en fil et se prolonge par une partie distale (132₃) de section supérieure à celle de la partie principale pour augmenter la surface d'appui du filtre contre la paroi du vaisseau.

4. Filtre selon la revendication 3, **caractérisé en ce que** les appendices (13₃a, 15₁, 15₂) sont constitués par des fils plats, tandis que la partie principale desdites pattes est constituée en fil rond.

5. Filtre sanguin expansible dans un vaisseau (31) d'un patient, sensiblement radialement vis-à-vis de l'axe dudit vaisseau, pour y retenir d'éventuels caillots de sang, le filtre présentant une extrémité proximale (3p) et une extrémité distale (3d), et comprenant :
-- des moyens de filtration qui comportent une tête (11) de laquelle sont issues des premières pattes (13₃a) expansibles radialement suivant sensiblement une première corolle, dans un état radialement non contraint,
-- des moyens (13₃b, 15₃) de maintien en place du filtre par rapport au vaisseau, ces moyens de maintien comprenant des secondes pattes (13₃b) radialement expansibles, s'étendant, depuis la tête du filtre vers l'extrémité proximale de celui-ci, sensiblement suivant une seconde corolle dans leur état radialement expansé, les première et seconde corolles étant disposées tête-bêche,
**caractérisé en ce que** certaines au moins des premières pattes (13₃a) comportent une partie principale (131₃) en fil raccordée à la tête (11) et qui se prolonge par une partie distale (132₃) de section supérieure, pour augmenter la surface d'appui du filtre contre la paroi du vaisseau.

6. Filtre selon la revendication 5, **caractérisé en ce que** la partie distale (132₃) est constituée par un fil plat.

7. Filtre selon la revendication 5 ou la revendication 6, **caractérisé en ce que** la partie distale (132₃) est constituée par un fil métallique plat.
